# EUROPEAN PATENT APPLICATION

(11) **EP 4 574 121 A1**
(43) Date of publication of application: **25.06.2025**
(21) Application number: 23218468.9
(22) Date of filing: 20.12.2023
(51) Int. Cl.: A61F 13/537

(54) **WASHABLE ABSORBENT HYGIENIC UNDERWEAR**

(71) Applicant: Essity Hygiene and Health Aktiebolag, 405 03 Göteborg (SE)
(72) Inventor: VASELL, Anna, 405 03 GÖTEBORG (SE); KALENTUN, Pia, 405 03 GÖTEBORG (SE); MAGNUSSON, Lisa, 405 03 GÖTEBORG (SE); HAGSON, Sandra, 405 03 GÖTEBORG (SE); LEANDER, Maja, 405 03 GÖTEBORG (SE)
(74) Representative: Nederlandsch Octrooibureau

(57) **Abstract**

Washable absorbent hygienic underwear (1) comprising an outer pant (10) and an absorbent assembly (100), being permanently secured in the outer pant (10) and comprising
- a liquid permeable layer (120),
- a liquid impermeable layer (150); and
- an absorbent member (140) located between the liquid permeable layer (120) and the liquid impermeable layer (150), the absorbent member (140) comprising a liquid acquisition structure (141) and a liquid retaining structure (142), the liquid acquisition structure (141) comprising or consisting of a spacer fabric (138), the liquid acquisition structure (141) being arranged between the liquid permeable layer (120) and the liquid retaining structure (142); and wherein the spacer fabric (138) completely overlaps with the liquid retaining structure (142) and has an area which is 65% or less of an area of the liquid retaining structure (142), the liquid retaining structure (142) having a density being higher than the density of the spacer fabric (138).

## Description

### TECHNICAL FIELD

The disclosure pertains to washable absorbent hygienic underwear comprising an outer pant and an absorbent assembly, the outer pant comprising a front portion, a rear portion and a crotch portion, the absorbent assembly being permanently secured in the outer pant and comprising a liquid permeable layer, a liquid impermeable layer and an absorbent member located between the liquid permeable layer and the liquid impermeable layer.

The washable absorbent hygienic underwear may be a pant-type incontinence protector for persons suffering from mild to moderate incontinence.

### BACKGROUND

Washable and reusable absorbent hygienic underwear as disclosed herein are intended for use by persons leading a normal social and physically active life and suffering from light to moderate urine incontinence.

It is a concern to provide such incontinence garments which are comfortable and wellfitting and which as much as possible resemble ordinary underwear. By necessity, incontinence protection garments include layers and materials which are not present in conventional underwear, such as a liquid permeable top layer which lets liquid pass through into at least one underlying liquid absorbent layer. It is also generally preferred that a liquid barrier layer is arranged on a garment-facing side of the absorbent material to prevent liquid from escaping through the incontinence protection underwear and to wet the user's outer garment.

The additional layers add bulkiness and stiffness to the underwear, making the underwear less conformable and comfortable to wear. A bulky non-conformable product may cause the wearer unease as a result of a real or perceived conspicuousness of the absorbent underwear.

The additional layers are joined to each other and to a pant-material by means of adhesives, stitched seams, etc. which may impart additional stiffness to parts of the underwear, particularly where multiple joins are arranged on top of each other. It is therefore a concern to arrange material layers in such a way that the flexibility of the underwear is decreased as little as possible and such that the part of the underwear which is provided for urine absorption is as slim and conformable as possible while offering adequate absorbency and leakage protection.

It is therefore an aim to optimize absorbent capacity and leakage protection while retaining comfort and conformability of the absorbent incontinence protection underwear as disclosed herein.

An object of the present disclosure is to offer an improved washable absorbent hygienic underwear, combining adequate absorbency and leak protection properties for light to moderate urine incontinence with a high likeness to ordinary washable hygienic underwear with regard to appearance and comfort.

### SUMMARY

The above and further objects may be achieved with washable absorbent hygienic underwear in accordance with claim 1. Variations of the disclosure are set out in the dependent claims and in the following description.

The disclosure concerns washable absorbent hygienic underwear comprising an outer pant and an absorbent assembly, the absorbent assembly and the outer pant having an extension in a longitudinal direction y, in a transversal direction x and in a thickness direction z perpendicular to the longitudinal direction y, and the transversal direction x, the outer pant comprising a front portion, a rear portion and a crotch portion, the absorbent assembly being permanently secured in the outer pant and comprising
- a liquid permeable layer,
- a liquid impermeable layer; and
- an absorbent member located between the liquid permeable layer and the liquid impermeable layer, the absorbent member comprising a liquid acquisition structure and a liquid retaining structure, the liquid acquisition structure comprising or consisting of a spacer fabric comprising a top web, a bottom web and an intermediate section comprising pile filaments extending in the thickness direction z and connecting the top web with the bottom web, the liquid acquisition structure being arranged between the liquid permeable layer and the liquid retaining structure; wherein
the spacer fabric completely overlaps with the liquid retaining structure and has an area which is 65% or less of an area of the liquid retaining structure, the liquid retaining structure having a density being higher than a density of the spacer fabric.

The outer pant is preferably a textile pant.

In the washable absorbent hygienic underwear as disclosed herein, the liquid retaining structure may have a basis weight which is higher than a basis weight of the spacer fabric.

In the washable absorbent hygienic underwear as disclosed herein, the liquid retaining capacity of the liquid retaining structure may be higher than the liquid retaining capacity of the spacer fabric.

In the washable absorbent hygienic underwear as disclosed herein, the absorbent assembly may be secured in the outer pant with the liquid acquisition structure being arranged in the crotch portion of the pant and/or in the front portion of the pant.

It is preferred that the liquid acquisition structure is arranged to cover an area corresponding to an expected fluid insult area of the washable absorbent hygienic underwear. In incontinence protection underwear for female users, the liquid acquisition structure is preferably arranged in the crotch portion of the outer pant and extends into the front portion of the pant and optionally also into the rear portion of the pant. The absorbent member may be divided in the longitudinal direction y into a front part, a rear part and a crotch part. The side edges of the absorbent member are preferably concavely curved so that the crotch part of the absorbent member is narrower than the front part and the rear part of the absorbent member. The crotch part preferably has a smaller width in the transverse direction x than a greatest width of the rear part of the absorbent member. The crotch part may have a smaller width than both the front part and the rear part of the absorbent member. The front part of the absorbent member preferably has a smaller width in the transverse direction x than the rear part of the absorbent member. A ratio between the greatest width of the front part of the absorbent member and the greatest width of the rear part of the absorbent member may be in the range of from 1:2 to 1:3.

The spacer fabric in the liquid acquisition structure may have a length along a longitudinal centerline L_{C} through the absorbent hygienic underwear which is smaller than a length of the absorbent member along the longitudinal centerline Lc. A ratio between the length of the spacer fabric in the liquid acquisition structure and the length of the absorbent member as measured along the longitudinal centerline L_{C} may be in the range of from 2.5:4 to 3:4. The spacer fabric in the liquid acquisition structure preferably has a front end edge positioned at the front end edge of the absorbent member and a rear end edge positioned at a distance d from the rear end edge of the absorbent member. The distance d is preferably at least 15% of a length lₐₘ of the absorbent member as measured along the longitudinal centerline L_{C}, the distance d preferably being in the range of from 15% to 40%, or 20% to 30%.

The spacer fabric in the liquid acquisition structure may have a shape in the x-y plane which coincides with the shape of the liquid retaining structure in the area of the absorbent member where the liquid acquisition structure is present.

However, other shapes are also contemplated for the spacer fabric in the liquid acquisition structure. A rectangular shape may be useful, as it is easy to produce without waste. A "funnel-shape", with a smaller width of the spacer fabric in the front part of the absorbent member than the width of the liquid retaining structure in the front part of the absorbent member may be beneficial as such arrangement renders the side portions of the absorbent member thinner, softer, and more flexible than in the area covered by the relatively stiffer spacer fabric in the liquid acquisition structure. The flexible, spacer fabric free, side portions of the absorbent member may bend and conform to the outer shape of the vulva of a female wearer of the absorbent hygienic underwear. A liquid retaining structure which extends outside of the periphery of the spacer fabric in the liquid acquisition structure may serve to mask the edges of the spacer fabric on an outer, garment-facing surface of the absorbent hygienic underwear, making the liquid acquisition structure less perceptible from the outside and less conspicuous when the absorbent hygienic underwear is worn under close-fitting clothing.

In the washable absorbent hygienic underwear as disclosed herein, the area of the s spacer fabric in the liquid acquisition structure may be 60% or less of an area of the absorbent assembly, such as 50% or less of the area of the absorbent assembly, such as 40% or less of the area of the absorbent assembly.

A technical benefit of this arrangement may include that that the relatively bulky spacer fabric which is used in the liquid acquisition structure is only arranged in an area of the absorbent assembly where a major part of liquid is expected to insult the washable absorbent hygienic underwear. As compared to the materials contemplated for the liquid retaining structure, the spacer fabrics contemplated for the liquid acquisition structure are relatively thick, bulky, stiff and compression resistant materials, which means that it is generally beneficial for reasons of comfort and wearer discretion to minimize the area of the absorbent hygienic underwear occupied by such material.

The liquid retaining structure may be arranged to provide supplementary absorbent material in areas of the washable absorbent underwear which may be subject to wetting but which are not part of a primary wetting and absorption zone being defined by the liquid acquisition structure. Such areas may include portions of the washable absorbent underwear located in the x-direction outside the primary wetting zone in the front portion of the washable absorbent underwear and/or a portion of the crotch portion of the washable absorbent underwear.

The washable absorbent underwear as disclosed herein is primarily intended for use by female incontinent persons and may be in the form of a female incontinence pant. In washable absorbent underwear for female users, the absorbent structure is typically placed with a major part of the absorbent material in the crotch portion and at the lower front portion of the underwear where initial wetting of the washable absorbent underwear is most likely to take place.

Furthermore, the washable absorbent underwear as disclosed herein may be a unisex washable absorbent underwear intended for use by male or female users.

Furthermore, the washable absorbent underwear as disclosed herein may also be a washable absorbent underwear suitable for absorbing other bodily fluids than urine fluid, such as for example absorbing menstrual fluids.

For all types of washable absorbent underwear as disclosed herein, the liquid retaining structure may have a larger area than the liquid acquisition structure and may be used to provide additional absorbency in areas of the washable absorbent underwear which are outside a primary wetting and absorption zone of the washable absorbent underwear, but which may still be subject to wetting.

Such wetting outside a primary wetting and absorption zone may be caused by sudden gushes of urine which overflows the liquid acquisition structure as well as by urine which happens to impact the washable absorbent hygienic underwear outside the primary wetting area. It has further been found that in washable textile absorbent underwear which free or substantially free from superabsorbent material, absorbed liquid has a higher migrate in the underwear under the influence of gravity. This means that the risk for leakage in the crotch portion may be higher than in a corresponding superabsorbent containing product. It may therefore be advantageous to provide additional absorbent capacity in the crotch portion, e.g., by the liquid retaining structure comprising one or more additional layer layers of absorbent material in the crotch portion of the washable absorbent hygienic underwear and/or by the liquid retaining structure forming leakage security zones along longitudinal side edges of the liquid acquisition structure in the crotch portion, to capture and absorb liquid running in the transverse direction out of the spacer fabric of the liquid acquisition structure.

The layers in the absorbent hygienic underwear as disclosed herein may be joined by means of seaming or welding. Construction seams are preferably flat seams, creating a smooth transition between material layers. The use of flat seams contributes to making the washable absorbent hygienic underwear as comfortable as possible.

In washable absorbent hygienic underwear as disclosed herein, the layers in the absorbent assembly may be connected with each other by a first construction seam and at least one second construction seam, each construction seam connecting a number of layers being less than the total number of layers in the absorbent assembly, a layer in the first construction seam being the same as a layer in the at least one construction seam and a layer in the first construction seam being different from a layer in the second construction seam.

A technical benefit of such an arrangement may include that no construction seam is made through all layers of the absorbent assembly, such that a compact seam area may be avoided.

By providing construction seams involving only some of the layers of the absorbent assembly where at least one layer is shared by the construction seams and at least one layer differs between the construction seams, the absorbent assembly is provided as a coherent unit in which two or more layers of the absorbent assembly are directly connected with each other and at least two layers are only indirectly connected to each other. Construction seams which are separated from each other in this manner may be applied to the absorbent assembly e.g., to provide a less conspicuous edge at the spacer material in the liquid acquisition structure. The divided construction seams, involving fewer layers of material will also be more flexible and conformable than a single seam through multiple layers.

The different material layers in the absorbent assembly are stitched together in zone transitions depending on which materials are present in each zone. A first construction seam may be arranged to connect layers of materials which are present only in a primary absorbent zone defined by the spacer fabric in the liquid acquisition structure to ascertain that the spacer fabric is securely attached in the absorbent assembly. A second construction seam or seams may be seams arranged at a peripheral edge of the absorbent assembly to hold the layers present there together and make it easier to apply a leak protection seal at the peripheral edge of the absorbent assembly.

In the washable absorbent hygienic underwear as disclosed herein, the spacer fabric may be secured to at least one other component in the absorbent member by means of a flat seam, such as a flatlock stitch. The at least one component may be the liquid retaining structure.

The spacer fabric may be secured to the at least one other component in the absorbent member by a flatlock stitch in class 600 according to the international standard ISO 4915, preferably by a flatlock stitch type 607 according to the international standard ISO 4915.

In the washable absorbent hygienic underwear as disclosed herein, the spacer fabric may be secured to the at least one other component in the absorbent member along a front-end edge and a rear-end edge of the spacer fabric and/or along a first side edge and a second side edge of the spacer fabric. Accordingly, the spacer fabric may be secured to the at least one other component in the absorbent member along a whole peripheral edge of the spacer fabric or only along selected portions of the peripheral edge of the spacer fabric. However, it may be preferred that all edges of the spacer fabric are secured, such as by a flat seam. A flat seam may serve to cover the edge of a relatively stiff spacer fabric and create a smoother and more comfortable area at the edge of the spacer fabric.

In the washable absorbent underwear as disclosed herein, the layer of spacer fabric may be in direct contact with the liquid retaining structure. Such arrangement may be beneficial as it promotes liquid transfer from the spacer fabric to the liquid retaining structure and facilitates rapid draining of the spacer fabric after a liquid insult.

The spacer fabric may alternatively, or in addition to being secured to the liquid retaining structure be secured to the liquid permeable layer. However, it may be beneficial to secure the spacer fabric only to the liquid retaining structure, as a seam between these two components may be concealed beneath the liquid permeable layer and will thereby be less conspicuous than a seam between the spacer fabric and the liquid permeable layer.

The spacer fabric in the liquid acquisition structure is a relatively stiff and noncompressible material. A high resistance to compression of the spacer fabric is beneficial as it allows the liquid acquisition structure to retain a large liquid-receiving and liquid containing space within the internal network formed by the pile filaments even when exposed to the compressive forces which normally arise during use of the underwear.

The spacer fabric in the liquid acquisition structure of the washable absorbent underwear may have a density of less than 0.1 g/cm³ at an applied pressure of 0.5 kPa. The spacer fabric in the liquid acquisition structure of the washable absorbent underwear may have a density of more than 0.03 g/cm³ at an applied pressure of 0.5 kPa. The density of the spacer fabric in the liquid acquisition structure of the washable absorbent underwear may be between 0.05 and 0.09 g/cm³ at an applied pressure of 0.5 kPa.

The spacer fabric may have a bulk of 10 g/cm³ or more at an applied pressure of 5 kPa.

The layer of spacer fabric may be in direct contact with the liquid permeable layer or may be in indirect contact with the liquid permeable layer by means of an intervening layer such as a reticulate textile layer or similar.

The density of the pile filament connections and their dimension makes the material soft but still able to withstand exerted pressures during its use. The spacer fabric expands almost immediately and completely to its original shape after the removal of the compressive force exerted during its use. "Density of the pile filament" is herein used as a measure of the number of pile filament connections per cm². The density of pile filament connections may be 50-150/cm², preferably 70-100/cm². The pile filaments may have a fineness of 80-130 dtex, preferably 90-120 dtex. The pile filaments may be of a monofilament yarn leading to a high compressive strength while keeping a low grammage.

The spacer fabric may have 5-15, preferably 8-12 courses/cm in the production direction of the fabric and 6-12, preferably 7-10 wales/cm in the cross direction of the fabric.

The bottom layer of the spacer fabric may have a denser structure with smaller openings than the top layer. Washable absorbent hygienic underwear according to any one of the preceding claims, wherein the liquid acquisition structure consists of a single layer of spacer fabric or of a layer of spacer fabric and one or more additional material layers. As disclosed herein, the liquid acquisition structure may contain further layers in addition to the spacer fabric layer, such as a second spacer fabric layer and/or an open-mesh material arranged between the liquid permeable layer and the spacer fabric.

In the washable absorbent underwear as disclosed herein, the layer of spacer fabric may be in direct contact with the liquid retaining structure. Such arrangement may be beneficial as it promotes liquid transfer from the spacer fabric to the liquid retaining structure and facilitates rapid draining of the spacer fabric after a liquid insult.

In the washable absorbent hygienic underwear as disclosed herein, the density of the liquid retaining structure may be 20%, 50% or 80% higher than the density of the spacer fabric.

The liquid acquisition structure drains liquid from the liquid permeable layer and acts as a temporary reservoir for the liquid until it has been absorbed by the underlying liquid retaining structure. The liquid may be distributed in the x and y directions by flowing in the internal space of the spacer fabric. In the liquid retaining structure, which preferably has higher density than the spacer fabric, liquid distribution is to a higher degree dependent on wicking in the capillaries formed between fibers in the structure. Due to the density difference, transportation of liquid from the high-bulk/low density liquid acquisition structure takes place quickly after a liquid insult, which means that the liquid acquisition structure returns to an air-filled lofty state within a relatively short time after wetting. Thereby, the liquid acquisition structure acts as an air-filled, pressure-resistant distance element between the liquid which is absorbed in the liquid retaining structure and a wearer's body. This is beneficial both as it provides the underwear with a less wet and clammy feeling and as the air-filled space in the spacer fabrics counteracts liquid return to the liquid permeable layer.

The spacer fabric in the washable absorbent underwear as disclosed herein may have a basis weight of 150 g/m² to 300 g/m² or a basis weight of 170 g/m² to 270 g/m².

In the washable absorbent underwear as disclosed herein, the liquid retaining capacity of the spacer fabric may be 0.2 g/cm² or less, and the liquid retaining capacity in the liquid retaining structure may be 0.3 g/cm² or more.

A particularly efficient combination of comfort properties and absorption and leakage protection properties has been found to be achieved with an absorbent assembly having the following features in combination: a liquid acquisition structure having a density of 0.1 g/cm³ or less, a basis weight of 300 g/m² or less, a liquid retaining capacity in the liquid acquisition structure of 0.2 g/m² or less and a liquid retaining capacity in the absorbent retaining structure of 0.3 g/cm² or more. The density of the liquid retaining structure is preferably at least 20% higher than the density of the spacer fabric in the liquid acquisition structure.

The liquid permeable layer may have a basis weight of less than 300 g/m² and the spacer fabric may have a density of less than 0.1 g/cm³, a basis weight of less than 300 g/m² and a thickness of 2.0 mm or more.

In the washable absorbent underwear as disclosed herein, the liquid retaining structure may consist of a single material layer or of two or more individual material layers. If the liquid retaining structure contains more than one layer of textile absorbent material, it may be beneficial that the layers provide a density gradient in the z-direction, with increasing density in the layers of the liquid retaining structure from a top layer facing the liquid permeable layer to a bottom layer facing the liquid impermeable layer.

In the washable absorbent hygienic underwear as disclosed herein, the absorbent assembly has a peripheral edge and the peripheral edge is sealed by a liquid impermeable edge seal.

The sealing strip may e.g., be adhesively applied to seal the edges of the material layers in the absorbent assembly. The sealing strip is liquid impermeable and preferably breathable and may be in the form of a polymer tape, such as a polyurethane tape. Alternatively, the peripheral edge of the absorbent assembly may be a "non-sealed" peripheral edge, having only a peripheral seam, referred to herein as being "fully stitched". However, combinations of sealed edge portions and stitched portions of the peripheral edge of the absorbent assembly are also contemplated for the washable absorbent hygienic underwear as disclosed herein. By way of example, the absorbent assembly may have sealed side edges in combination with stitched front and rear end edges or sealed side edges, a sealed rear edge and a stitched front edge. The sealed edges are generally thicker and less flexible than the edges which are stitched, which means that it may be desirable to arrange sealed edges only where there is a higher risk of leakage, such as along the side edges of the absorbent assembly.

By *washable* as defined herein is implied an ability of the absorbent hygienic underwear to withstand laundering at 40 °C at least 100 times without any appreciable loss in appearance, integrity and/or functionality. When used herein laundering means that the absorbent underwear may be subjected to an aqueous solution containing detergent.

As used herein, the term *absorbent underwear* refers to garments which are worn as a pair of underpants and which are intended to be placed against the skin of the wearer to absorb and contain body fluids such as urine. The incontinence underwear as disclosed herein is an undergarment, or underpant intended for use by persons suffering from light to moderate incontinence. The absorbent underwear is a fabric underwear, also referred to as a textile underwear, and is not a diaper. The underwear according to the present disclosure is intended to be worn in the same manner as conventional underwear and to be laundered after use for reuse as an absorbent incontinent protection underwear.

The term *fabric* as used in the present disclosure refers to webs of single or multiple layers of textile materials. The fabric may be knitted or woven wherein knitting is a method of constructing a fabric by interlocking a series of loops of one or more yarns. There are two major classes of knitting namely warp and weft knitting. Weaving is a method or process of interlacing yarns so that they cross each other at right angles to produce woven fabric. The warp yarns run lengthwise in the fabric and the filling threads (weft) or picks, run from side to side.

The term *fiber* is used herein in a broad sense and includes fibers which have a measurable length, such as staple fibers, as well as filaments which do not have a measurable length and which are often referred to as being *"endless".* The term *fiber* is also intended to cover microfibers and fibrils. The fibers in the textile materials which are used in the washable absorbent hygienic underwear as disclosed herein may be spun into yarns or threads, each yarn or thread comprising multiple fibers.

When used herein a *permanent attachment,* refers to an attachment which is intended to remain intact and not be broken before, during or after use of the washable absorbent hygienic underwear as disclosed herein. A permanently attached absorbent assembly is an absorbent assembly which cannot be separated from the outer pant of the washable absorbent hygienic underwear without breaking or otherwise destroying the washable absorbent underwear. The absorbent assembly is not necessarily directly bonded to the outer pant but may instead be attached via leg opening seams or bonded by an intermediate means such as a sealing tape.

The washable absorbent assembly may contain no superabsorbent particles. However, the washable absorbent assembly may contain superabsorbent fibers in the liquid retention structure.

The spacer fabric may contain absorbent cellulosic fibers in the top and bottom layers or may be free or substantially free from absorbing fibers. The spacer fabric does not contain superabsorbent material of any kind. It has further been found that in washable textile absorbent hygienic underwear which does not include superabsorbent material, the liquid has a higher tendency to transfer downwards under the influence of gravity. This means that the risk for leakage in the crotch portion of the incontinence underwear is higher than in a superabsorbent containing product.

The absorbent assembly comprises the following components, as seen in a direction from an inner wearer-facing surface of the washable absorbent hygienic underwear towards an outer garment facing surface of the washable absorbent hygienic underwear:

### A liquid permeable layer

The liquid permeable layer is a liquid wicking and transfer layer. The liquid permeable layer is preferably a hydrophilic layer which can attract aqueous liquid and let the liquid pass through the layer to underlying layers of the absorbent assembly, such as an underlying liquid acquisition layer which can drain liquid from the liquid permeable layer.

The liquid permeable layer may be made from any suitable material which can withstand repeated washing. Suitable textile materials include fibrous knitted or woven materials, as well as mesh materials, such as polymer netting, perforated plastic materials, and the like.

The layer may comprise or consist of natural fibers such as cotton, regenerated cellulose fibers such as viscose fibers or synthetic fibers such as polyester fibers, polyolefin fibers, etc. The synthetic fibers may be mono-, bi-, or multi-component fibers. Different types of fibers may be combined in the layer, e.g., to promote liquid wicking through the layer while at the same time retaining a dry surface feeling. It may be preferred that the liquid permeable layer comprises at least a portion of inherently wettable (hydrophilic) fibers which may promote liquid uptake and wicking in the layer. The liquid permeable layer may comprise at least a portion of synthetic fibers which do not absorb or retain liquid. The layer may, for example, comprise or consist of a combination of viscose or other regenerated fibers and polyolefin fibers, such as polypropylene fibers. However, liquid permeable layers made exclusively of natural or regenerated cellulosic fibers as well as liquid permeable layers made exclusively of synthetic fibers are also contemplated for the washable absorbent hygienic underwear as disclosed herein. Unless having a great open area, a liquid permeable layer made exclusively from synthetic, inherently non-wettable fibers will generally be treated, such as by a surfactant, to render the outer surface of the fibers hydrophilic and wettable.

### A liquid acquisition structure

The liquid acquisition structure drains liquid from the liquid permeable layer, distributes liquid in the liquid acquisition structure and transfers liquid to an underlying liquid absorbing layer, such as a liquid retaining structure. The liquid acquisition structure preferably has a high bulk and an internal volume which allows liquid to be received and temporarily held in the layer. The liquid acquisition structure may further serve as a spacer element, isolating liquid from a wearer's skin and preventing absorbed liquid from returning to the liquid permeable layer also when the washable absorbent hygienic underwear is exposed to pressure during use.

The liquid acquisition structure may be composed of one or more layers of woven, knitted or nonwoven textile material, such as a three-dimensional spacer material, a terry cloth material or washable nonwoven material.

As disclosed herein, the liquid acquisition structure used in the washable absorbent hygienic underwear may preferably comprise or consist of a spacer fabric. The spacer fabric comprises two outer layers, such as two knitted layers which are connected to each other by spacer elements extending generally perpendicular between the layers. The spacer elements are formed from pile filaments which are relatively stiff and resilient as compared to the fibers in the outer layers.

The spacer fabric may be produced by knitting, as an integral structure. The outer layers may comprise wettable, absorbent fibers, such as natural or regenerated cellulosic fibers and the spacer elements may be made from synthetic material which will retain its resiliency in a wet state.

When the washable absorbent hygienic underwear is being used, urine which passes through the liquid permeable layer and into the liquid acquisition structure may be absorbed by the outer layers of the spacer fabric. The non-absorbent pile filaments create a liquid receiving and containing space between the outer layers of the spacer fabric. The liquid receiving and containing space is also referred to herein as an intermediate layer of the spacer fabric. The liquid containing space may serve as a reservoir for liquid which may be subsequently absorbed by the lower of the two outer layers and of further absorbent material arranged below the liquid acquisition structure. Liquid may run relatively freely within the space of the channel network formed by the pile filaments between the outer layers of the spacer material, allowing the liquid to spread out over a greater area of the absorbent structure and increasing the area of the absorbent structure over which liquid transfer to a lower layer or layers may take place.

### A liquid retaining structure

The liquid retaining structure may be composed of one or more layers of woven, knitted or nonwoven textile material arranged to receive liquid from the liquid acquisition structure.

The liquid retaining structure may be made from a three-dimensional spacer material, a terry cloth material or washable nonwoven material. The liquid retaining structure preferably has a higher ability of absorbing and retaining liquid in the structure than the liquid acquisition structure and can drain liquid from the liquid acquisition structure.

The liquid retaining structure may comprise synthetic fibers, natural fibers or man-made cellulosic fibers as set out above for the liquid acquisition structure. The liquid retaining structure may contain super absorbent fibers. The liquid retaining structure may comprise any blend or combination of suitable fibers.

### A liquid impermeable layer

The liquid impermeable layer serves to keep liquid in the absorbent member of the absorbent assembly and to prevent liquid from leaking out through the outer pant or being absorbed by the outer pant material.

The liquid impermeable layer is typically a polymer film layer or membrane. The polymer film is preferably breathable, letting air and water vapor pass through the film but forming a liquid barrier. A breathable polyurethane film may be used for the liquid impermeable layer. The liquid impermeable layer may be laminated to an absorbent layer such as a layer of the liquid retaining structure, such as by adhesive or by being extruded onto the absorbent layer. The adhesive may be a polyurethane based adhesive.

The absorbent assembly may further include one or more sealing strips. The sealing strips may e.g., be adhesively applied to cover a seam connecting first and second panels of a three-dimensionally shaped absorbent assembly and/or may be applied to seal cut edges of the material layers in the absorbent assembly. The sealing strips are liquid impermeable and preferably breathable and may be in the form of a polymer tape, such as a polyurethane tape.

The material in the outer pant is preferably a knitted material such as a cotton knitted material comprising elastic material such as elastane. The washable absorbent hygienic underwear is preferably provided with a separately applied elastic waistband. The elastic waistband may comprise the same knitted material as the outer pant material, or any other suitable textile material. The elastic waistband preferably comprises waist elastic elements, such as elastic threads, or one or more elastic bands.

The liquid permeable layer, the absorbent layer or layers and the liquid impermeable layer of the absorbent assembly may be attached to the outer pant by means of seaming. A liquid impermeable tape may be applied around all or part of the peripheral edges of the absorbent assembly.

### BRIEF DESCRIPTION OF THE DRAWINGS

The washable absorbent hygienic underwear as disclosed herein will be further explained hereinafter by means of non-limiting examples and with reference to the appended drawings wherein:
- Fig. 1: shows a perspective frontal view of absorbent underwear for female users;
- Fig. 2: shows the absorbent underwear of Fig. 1, from an inner, wearerfacing side, and in a flat-out state with open side seams;
- Fig. 3: shows a cross section taken along the line III-III in Fig. 2;
- Fig. 4a-4c: show different configurations of absorbent assemblies in absorbent hygienic underwear as disclosed herein;
- Fig. 5: shows a perspective view of an exemplary spacer fabric;
- Figs. 6: shows an exploded cross sectional view along the line VI-VI of the absorbent assembly 100 shown in Fig. 4a with the position of constructional seams indicated.

### DETAILED DESCRIPTION

It is to be understood that the drawings are schematic and that individual components or features, such as layers of material are not necessarily drawn to scale. The absorbent hygienic underwear shown in the figures is provided as an example only and should not be considered limiting to the invention as disclosed herein.

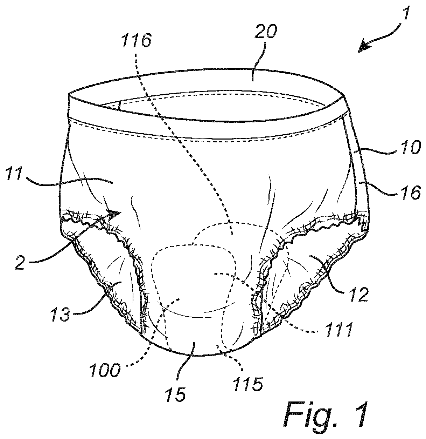

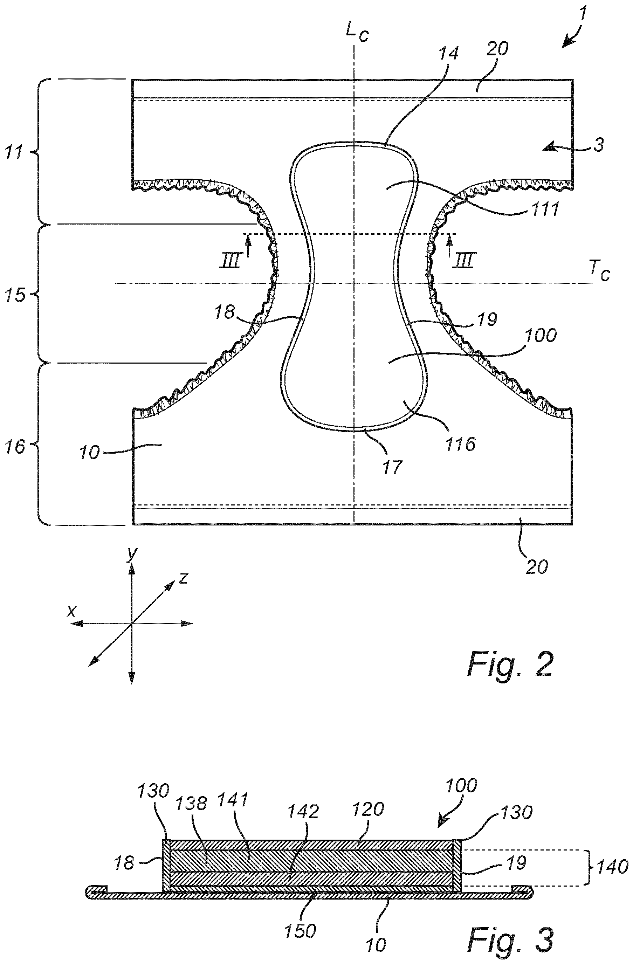

The washable absorbent hygienic underwear disclosed herein should not be construed as being limited to the aspects set forth herein but can be varied within the scope of the appended claims. Although the washable absorbent hygienic underwear shown in Figs. 1 and 2 takes the form of incontinence panties for female users, it is to be understood that the washable absorbent underwear may be unisex incontinence underwear. The washable absorbent underwear may be of any useful design, such as hipster panties, briefs, boxer panties, etc., as known in the art. The washable absorbent hygienic underwear shown in Figs. 1 and 2 takes the form of incontinence panties for female users. However, it is to be understood that the washable absorbent underwear may be also for other body liquids than urine, such as for example menses.

Fig. 1 shows a frontal perspective view of absorbent underwear 1 as seen from the outer, garment-facing side 2. The absorbent underwear 1 in Fig. 1 is an incontinence pant for female incontinent persons.

Fig. 2 shows the underwear 1 of Fig. 1 from the inner, wearer-facing side 3 in a flat-out state with open side seams.

The absorbent underwear 1 comprises an outer pant 10 of textile material and includes an absorbent assembly 100 which is permanently secured in the outer pant 10, e.g., by being sewn onto the outer pant 10 and/or by being adhesively attached to the outer pant 10.

As shown in Fig. 2, the outer pant 10, the whole absorbent underwear 1 and the absorbent assembly 100 have an extension in a longitudinal direction y, in a transverse direction x and in a thickness direction z which is perpendicular to the longitudinal direction and the transverse direction. The absorbent underwear 1 has a front portion 11, a rear portion 16 and a crotch portion 15 bridging the front and rear portions 11,16.

The absorbent underwear 1 which is shown in Figs. 1 and 2 includes an elastic waistband 20 for providing improved retention of the absorbent underwear 1 on a wearer's body. An elastic waistband is an optional component of the absorbent underwear 1 as disclosed herein and may be applied as a separate component to the outer pant 10 of the absorbent underwear 1 or may be integrally formed with the outer pant 10, e.g., as a portion of the outer pant 10 having increased elasticity as compared to other parts of the outer pant 10. In the first case, the waistband may be any type of waistband as used in conventional underwear, such as a waistband made from cotton and elastane. In the latter case, the textile material in the waistband is typically the same as in other parts of the outer pant 10, such as cotton. A waistband which is integral with the outer pant 10 may be formed by folding over an edge portion of the outer pant 10 onto itself to form a waistband comprising two layers of pant material. Another way of producing a distinct integral waistband is by having a smaller textile gauge at the waist portion of the outer pant. The waistband may comprise additional elastic elements to provide enhanced elasticity at the waist portion of the outer pant.

The outer pant 10 may further include leg cuffs arranged around the leg openings 13, 12of the pant. The leg cuffs may be elasticated leg cuffs comprising additional elastic elements providing enhanced elasticity at the leg openings 13,14.

The material of the outer pant 10 is typically a conventional underwear material such as a knitted material and may include or consist of naturally derived fibers, man-made fibers and mixtures of different fibers. Useful naturally derived fibers include cotton, wool, silk, cellulose, regenerated cellulose, rayon, viscose, modal, lyocell, tencel, bamboo, hemp, flax, ramie, coir, or banana.

Useful manmade synthetic fibers include polyamide, acrylic, polyester. As set out herein, the textile material may comprise a mixture or combination of naturally derived and/or synthetic fibers. The fibers may be recycled fibers. The textile material may comprise elastically stretchable material, e.g., elastane filaments, so that the absorbent underwear can conform to the wearer's body and adapt to the wearer's movements. The elastic conformability of the outer pant contributes to preventing liquid from leaking out through the leg openings and to keep the absorbent assembly in place against the wearer's body. A textile material is generally breathable, allowing vapor to escape from the wearer's skin and from the absorbent assembly. Advantageously, the material in the outer pant is a combination of cotton and elastane.

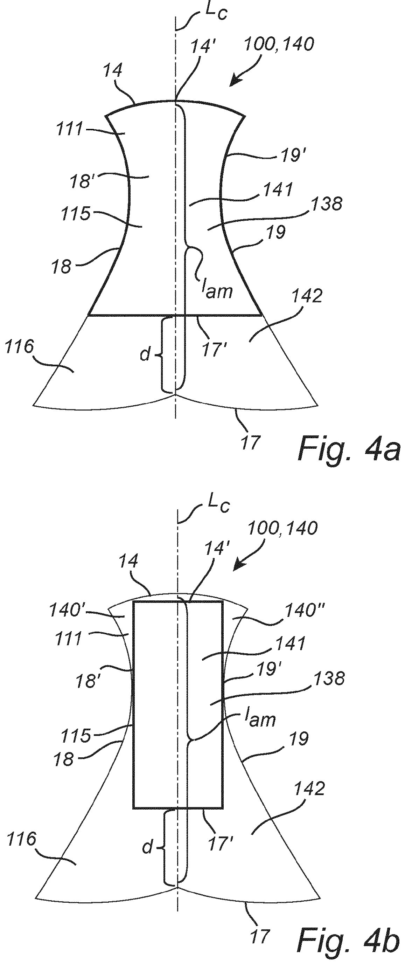

The absorbent assembly 100 is integrated with the outer pant 10 and is positioned in a region of the outer pant 10 mostly likely to be exposed to micturition to provide liquid acquisition, liquid retaining capacity and leakage protection. In the absorbent underwear 1 shown in Figs. 1 and 2, the absorbent assembly is hourglass shaped and is positioned generally on a longitudinal centerline L_{C} and a transverse centerline T_{C}. The absorbent assembly 100 is present in the crotch portion 15 and has a front part 111 extending into the front portion 11 of the absorbent underwear 1, a rear part 116 extending into the rear portion 16 of the absorbent underwear 1 and a crotch part 115 between the front part 111 and the rear part 116 of the absorbent assembly 100. With further reference to the example shown in Figs. 1 and 2, the absorbent assembly 100 has a generally hourglass shape with rounded front and rear edges 14, 17 and generally longitudinally extended concave side edges 18, 19. However, it is to be understood that the absorbent assembly 100 may have any useful shape within the scope of protection of the claims, such as the shapes shown in Figs. 4a-4c.

As disclosed herein, the absorbent assembly 100 comprises several layers of different materials arranged stacked on each other to form an absorbent member 140 capable of quickly absorbing and storing discharged urine. With reference to Fig. 3, the absorbent assembly 100 comprises a liquid permeable layer 120, a liquid impermeable layer 150 and the absorbent member 140 which is arranged between the liquid permeable layer 120 and the liquid impermeable layer 150. The absorbent structure 140 comprises a liquid acquisition structure 141 comprising a spacer fabric and a liquid retaining structure 142 of textile absorbent material. It is to be understood that the liquid acquisition structure 141 of textile absorbent material and the liquid retaining structure 142 of textile absorbent material may each comprise two or more sublayers or strata.

As best seen in Fig. 3, the liquid acquisition structure 141 is arranged between the liquid permeable layer 120 and the liquid retaining structure 142. As set out herein, the liquid permeable layer 120 is a textile liquid permeable layer having a basis weight of 300 g/m² or less. The liquid retaining structure 142 is an absorbent component which may consist of a single layer of absorbent textile material or may comprise 2 or more individual layers of absorbent textile material.

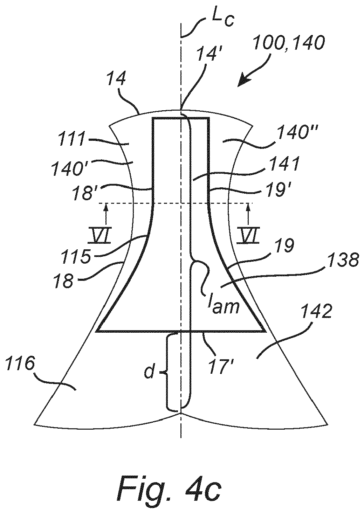

With reference now to Figs. 4a-4c, there is shown a number of different configurations of absorbent assemblies 100 in washable absorbent hygienic underwear 1 as disclosed herein. In the absorbent assemblies 100 as disclosed herein, it is preferred that the liquid acquisition structure 141 is arranged to cover an area corresponding to an expected fluid insult area of the washable absorbent hygienic underwear, also referred to herein as a primary absorption zone. In incontinence protection underwear for female users, the liquid acquisition structure is preferably arranged in the crotch portion 15 of the outer pant 10 and extends into the front and rear portions 11,16 of the pant 10. As shown in Fig. 2, the absorbent assembly 100 and the absorbent member 140 may be divided in the longitudinal direction y into a front part 111, a rear part 116 and a crotch part 115. The side edges 18, 19 of the absorbent member 140 may be concavely curved so that the crotch part 115 of the absorbent member 140 is narrower than the front part 111 and the rear part 116 of the absorbent member 140 and has a greatest width in the transverse direction x which is smaller than a greatest width of both the front part 111 and the rear part 116 of the absorbent member 140 as shown in Figs. 2 and 4a. The front part 111 of the absorbent member 140 may have a smaller width in the transverse direction x than the rear part 116 of the absorbent member 140, as shown in Figs. 2, 4a and 4c. A ratio between the greatest width of the front part 111 of the absorbent member 140 and the greatest width of the rear part 116 of the absorbent member 140 may be in the range of from 1:2 to 1:3.

The spacer fabric in the liquid acquisition structure 141 may have a length along a longitudinal centerline L_{C} through the absorbent hygienic underwear which is smaller than a length of the absorbent member 140 along the longitudinal centerline Lc. A ratio between the length of the spacer fabric in the liquid acquisition structure 141 and the length of the absorbent member 140 as measured along the longitudinal centerline L_{C} may be in the range of from 2.5:4 to 3:4. The spacer fabric 138 in the liquid acquisition structure 141 has first and second side edges 18', 19' extending generally in the longitudinal direction y. The spacer fabric 138 in the liquid acquisition structure 141 preferably has a front end edge 14' positioned at the front end edge 14 of the absorbent member 140 and a rear end edge 17' positioned at a distance d from the rear end edge 17 of the absorbent member 140, as seen in Figs. 4a-4c. The distance d is preferably at least 15% of a length lₐₘ of the absorbent member as measured along the longitudinal centerline L_{C}, the distance d may be in the range of from 15% to 40%, or in the range from 20% to 30%.

The spacer fabric in the liquid acquisition structure 141 may have a shape in the x-y plane which coincides with the shape of the liquid retaining structure in the area of the absorbent member where the liquid acquisition structure is present, as illustrated by Fig. 4a where the spacer fabric in the liquid acquisition structure 141 completely coincides with the liquid retaining structure 142 in the front and crotch parts 111, 115 of the absorbent member 140 and also with a small portion of the rear part 116 of the absorbent member 140 into which the spacer fabric in the liquid acquisition structure 141 extends.

However, other shapes are also contemplated for the spacer fabric in the liquid acquisition structure 141. A rectangular shape as shown in Fig. 4b may be useful, as it is easy to produce without waste. A "funnel-shape" as shown in Fig. 4c with a smaller width of the spacer fabric in the front part 111 of the absorbent member 140 than the width of the liquid retaining structure 142 in the front part 111 of the absorbent member 140 may be beneficial as such arrangement renders the side portions 140', 140" of the absorbent member 140 thinner, softer, and more flexible than in the area covered by the relatively stiffer spacer fabric in the liquid acquisition structure 141. The flexible, spacer fabric free, side portions 140', 140" of the absorbent member 140 may bend and conform to the outer shape of the vulva of a female wearer of the absorbent hygienic underwear 1. A liquid retaining structure 142 which extends outside of the periphery of the spacer fabric in the liquid acquisition structure 141 may serve to mask the edges of the spacer fabric on an outer, garment-facing surface of the absorbent hygienic underwear, making the liquid acquisition structure less perceptible from the outside and less conspicuous when the absorbent hygienic underwear is worn under close-fitting clothing.

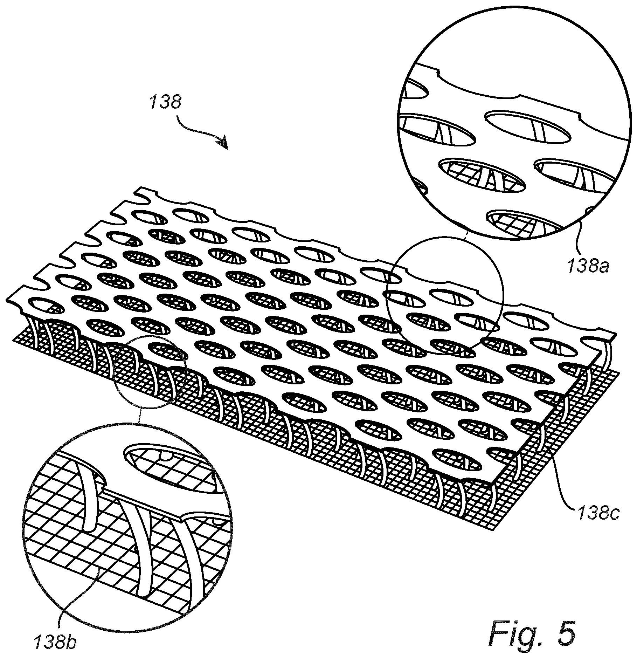

The liquid acquisition structure 141 comprises or consists of a spacer fabric 138 as shown in Fig. 5 where the liquid acquisition structure 141 is represented by a single layer of spacer fabric 138. As disclosed herein, the liquid acquisition structure 141 may contain further layers in addition to the spacer fabric layer, such as a second spacer fabric layer and/or an open-mesh material arranged between top layer 120 and the spacer fabric 130.

The spacer fabric 138 comprises a top layer 138a and a bottom web 138b and an interconnecting section 138c of pile filaments extending between the top web 138a and the bottom web 138b. The top web 138a and the bottom web 138b of the spacer fabric 138 may have different configurations to control the flow of the liquid in the spacer fabric 130 and liquid transfer to the liquid retaining structure 142. As disclosed herein, the top web 138a may have a lower density than the bottom web 138b. Furthermore, the density of the top web 138a and the bottom web 138b are higher than the density of the intermediate section 138c.

The liquid retaining capacity of the spacer fabric 138 is preferably considerably lower than the liquid retaining capacity of the liquid retaining structure 142. Accordingly, the liquid retaining capacity of the spacer fabric may be 0.2 g/cm² or less and the liquid retaining capacity in the liquid retaining structure 142 may be 0.3 g/cm² or more.

The density of the liquid retaining structure 142 is preferably considerably higher than the density of the spacer fabric 138 in the liquid acquisition structure 141. Accordingly, the density of the liquid retaining structure 142 may be 20% higher than the density of the spacer fabric 138, or more than 50% higher than the density of the spacer fabric 138.

The spacer fabrics 138 which are useful in a liquid acquisition structure 141 as disclosed herein have a density of 0.1 g/cm³ or less and a thickness of 2 mm or more and have a basis weight and a density which are lower than the basis weight and density of the liquid retaining structure 142.

A spacer fabric 138 is more compression resistant than other components that may be used for liquid acquisition structures in absorbent articles but at the same time has a relatively low density. This allows for a slim and efficient liquid acquisition structure that will maintain its structure and fluid flow control properties when pressure is exerted on it during use of the washable absorbent hygienic underwear 1. As set out herein, the spacer fabric preferably has a bulk (ratio thickness/basis weight) of 10 cm³/g or more at an applied pressure of 0.5 kPa, and also at an applied pressure of 5 kPa.

The number of pile filament connections per cm² and the dimension of the pile filaments makes the material soft but still able to withstand exerted pressures during its use. The spacer fabric 138 is pressure resilient and expands immediately and almost completely to its original shape after the removal of a compressive force exerted during its use.

The pile filaments 138c may together form channels, preferably in a diagonal direction of the fabric, creating an internal channel network promoting efficient distribution of liquid in the liquid acquisition structure 141.

The spacer fabric 138 is a highly porous material in which a free volume is present, also when the absorbent structure 140 is exposed to pressure exerted by a user wearing the absorbent hygienic underwear 1, as a result of high resistance to compression. Due to the free volume, the spacer fabric 138 can receive and temporarily hold a relatively large liquid volume. Thus, body liquids discharged into the absorbent assembly 100 can be effectively transferred from the liquid permeable layer 120 into to the spacer fabric 138 and can flow in the internal channel network in the intermediate section 138c of the spacer fabric 138 to be distributed over the area of the spacer fabric 138. The distributed liquid can then be transferred to the liquid retaining structure 142 over an area which may be as large as the area of the spacer fabric 138. The liquid which is transferred to the liquid retaining structure 142 is absorbed and retained in the absorbent material of the liquid retaining structure 142.

The top web 138a of the spacer fabric 138 may have an open structure to ensure rapid inflow and effective distribution of liquid in the spacer fabric 138. The spacer fabric 138 is preferably substantially free from absorbing fibers and does not contain superabsorbent material. However, the top web 138a and the bottom web 138b may contain hydrophilic fibers, such as regenerated cellulose fibers to promote wettability and liquid transfer between the liquid permeable layer 120 and the top web 138a and the bottom web 138b and the liquid retaining structure 142.

The spacer fabric 138 preferably comprises thermoplastic polymer fibres, preferably selected from but not limited to, polyesters, polyamides and polyolefins such as polyethylenes and polypropylenes, and may be a mixture or combination of any of these. The spacer fabric 138 may also advantageously comprise fibres from cotton and/or viscose. For a preferred spacer fabric 138, the yarns of the top and bottom webs 138a, 138b as well as the pile filaments 138c are of polyester.

The spacer fabric 138 may also contain surfactant to facilitate liquid penetration into the spacer fabric 138. It is also desirable that the spacer fabric 138 can be quickly drained from liquid by the liquid retaining structure 142, thus restoring free volume capacity for a next gush of urine.

The spacer fabric 138 is arranged in the absorbent assembly 100 with the top web 138a of the spacer fabric 138 facing and preferably in direct contact with the liquid permeable layer 120. The bottom web 138b of the spacer fabric 138 is facing the liquid impermeable layer 150 and is preferably in direct contact with the liquid retaining structure 142.

The liquid impermeable layer 150 is typically a polymer film layer or membrane and is preferably breathable, letting air and water vapor pass through but forming a barrier against liquid penetration. The liquid impermeable layer 150 may be directly attached to an absorbent layer, such as an absorbent layer in the liquid retaining structure 142. The attachment may be by a seam or by lamination. Lamination of the liquid impermeable layer 150 to the liquid retaining structure 142 may be made by means of a construction adhesive or by extruding the liquid permeable layer 150 onto the absorbent layer of the liquid retaining structure. The construction adhesive may be a polyurethane based adhesive.

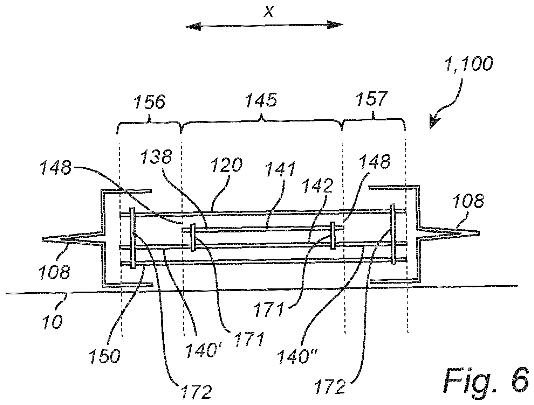

Fig. 6 shows a cross-section along the line VI-VI of the absorbent assembly 100 shown in Fig. 4a and with the absorbent assembly 100 attached in an outer pant 10 of washable absorbent hygienic underwear 1 as disclosed herein. The liquid acquisition structure 141 is represented by a single layer of spacer fabric, which is arranged between the liquid retaining structure 142 and the liquid permeable layer 120. The spacer fabric has a smaller extension in the transverse direction x than the liquid retaining structure 142. The liquid retaining structure 142 has the same extension in the transverse direction x as the liquid permeable layer 120 and the liquid impermeable layer 150 and extends in the transverse direction x outside of a primary absorbent zone 145 defined by the spacer fabric in the liquid acquisition structure 141, thereby forming thinner side portions 140', 140" of the absorbent member 140. The liquid permeable layer 120, the liquid impermeable layer 150 and the liquid retaining structure in the side portions 140', 140" of the absorbent member 140 outside the periphery 148 of the primary absorbent zone 145 form secondary absorbent zones 156, 157 of the absorbent assembly 100.

The primary absorbent zone 145 may also be referred to as a liquid storage zone. The liquid storage zone will always contain the spacer fabric in the liquid acquisition structure 141 and a liquid retaining structure142 comprising at least one layer of textile absorbent material. The secondary absorbent zones 156, 157 of the absorbent assembly 100 may also be referred to as leakage security zones, providing supplementary absorption capacity, and a barrier against leakage out to the outer pant 10.

As set out herein, the provision of one or more secondary absorbent zones 156, 157 may serve to create a soft, less perceptible, and conspicuous transition at the periphery 148 of the primary absorbent zone 148. In absorbent assemblies 100, such as the absorbent assembly in Fig. 6, where the spacer fabric in the liquid acquisition structure 141 is a relatively stiff and thick material, the edge masking effect of the liquid retaining structure 142 may be particularly beneficial. Any portions 140', 140" of the liquid retaining structure 142 forming secondary absorbent zones 156, 157 of the absorbent assembly 100 are preferably thinner and more flexible than the spacer fabric in the primary absorbent zone 145. The primary absorbent zone 145 is a target absorbent area of the absorbent structure 140 in the absorbent assembly 100 and the secondary absorbent zone or zones 156, 157 may be considered to be back-up absorbent areas which can absorb and retain liquid which either impinges the absorbent assembly outside of the primary absorbent zone 145, or which moves away from the primary absorbent zone 145, e.g., under the influence of gravity.

As further illustrated by Fig. 6, the layers or structures 120, 141, 142, 150 in the absorbent assembly 100 of absorbent hygienic underwear 1 as disclosed herein are preferably connected with each other by a first construction seam 171 and at least one second construction seam 172. Each construction seam connects a number of layers which is less than the total number of layers in the absorbent assembly 100. At least one layer in the first construction seam is different from a layer in the second construction seam, the absorbent assembly 100 being formed into a coherent component by at least one layer in the first construction seam 171 being the same as a layer in the second construction seam 172. Accordingly, no construction seam 171, 172 involves all layers of the absorbent assembly 100 but shares at least one layer with another construction seam 171, 172. Every layer in the absorbent assembly 100 is directly connected to at least one other layer in the absorbent assembly 100 and is directly or indirectly connected to all other layers in the absorbent assembly 100.

In Fig. 6, the spacer fabric in the liquid acquisition structure 141 is attached to the liquid retaining structure 142 by a first construction seam 171. The liquid retaining structure 142 is attached to the liquid permeable layer 120 and to the liquid impermeable layer 150 by a second construction seam 172, the first and second construction seams 171, 172 being non-overlapping seams and being placed at a distance from each other in the x-y plane.

The construction seams 171, 172 are preferably flat seams and may be made by a flatlock stitch. The flatlock stitch may be a flatlock stitch in class 600 according to the international standard ISO 4915, such as a flatlock stitch type 607 according to the international standard ISO 4915.

Flatlock seams are formed from groups of threads; needle-, cover-, and looper threads, to create strong, band-shaped and flat seams. The needle threads extend back and forth through the front and back sides of the layer or layers of fabric, while the cover thread extends back and forth across the width of the seam only on the front side, and the looper thread extends back and forth across the width of the seam only on the back side of the fabric. Flatlock seams are advantageously used for next-to-the-skin garments such as e.g., underwear since these garments must be extra comfortable, so they don't chafe the skin or cause any other discomfort. Absorbent material such as e.g., the spacer material 138 described herein typically benefits from being joined by flatlock seams because a flatlock seam generally causes less compaction of the material than conventional seams which means that the flatlock seams are softer and more flexible than conventional seams and that an internal void structure of the material may be retained to a higher degree than when using conventional seams.

### EXAMPLES AND DESCRIPTION OF TEST METHODS

### Example 1 - Density measurement

The weight and the thickness of the sample were measured. The thickness of the material was measured according to a standard test method NWSP120.6, option A, at 0.5 kPa pressure. The basis weight was calculated by dividing the weight with the area. Then, the density was calculated by dividing the basis weight with the thickness.

The density and basis weight have been measured on following material samples:

| Sample | Description | Density (g/cm³) | Basis weight (g/m²) |
|---|---|---|---|
| A | Spacer fabric | 0.06 | 247 |
| B | Spacer fabric | 0.06 | 175 |
| C | Spacer fabric | 0.08 | 230 |
| D | Ref spacer fabric | 0.13 | 258 |
| E | Ref spacer fabric | 0.18 | 400 |
| F | Abs retaining structure | 0.12 | 330 |
| G | Liquid permeable layer | 0.15 | 220 |

Sample A, B and C are spacer fabric materials from Muller Textil.

Sample D is a reference spacer fabric material from a commercially available washable absorbent underwear named Confitex Full Brief Extra, size M. Sample E is a reference spacer fabric from a commercially available washable absorbent underwear named ProTech Dry women, size M. Sample F is an absorbent retaining structure of terry material. Sample G is a liquid permeable layer of polyester.

### Example 2 - Compression resistance measurement

Different spacer fabric materials have been compression tested, to compare the compression resistance of spacer fabric in commercially available washable absorbent underwear with spacer fabrics of the present invention. Sample A, B and C are three spacer fabrics according to the invention. Sample D and E are two reference spacer fabrics from commercially available washable absorbent underwear.

The principle of the method is to slowly compress a material with a metal rod to a force of 62 N while continuously measuring the thickness of the material. The result consists of the data points for force and extension. The force translates to a pressure given the contact area of the rod. The foot of the metal rod/piston has a diameter of 35 mm. The rod is mounted in a 100 N load cell in the upper fixture of an Instron testing apparatus. A flat plate is mounted in the bottom fixture and is centered under the rod so that a sample may be placed on top of the plate and be compressed without movement of the plate. The rate of movement of the rod is 10 mm per minute.

To test a sample, the rod is moved manually so that it is above the surface of the sample and the program is started. The rod moves down at a speed of 10 mm per minute until the limiting force is reached.

The sample has an area of 10 cm² and is punched from the material. The rod is pressed over the centre of the sample and each sample is tested two times with 10 min between the first and second test to allow the material to recover.

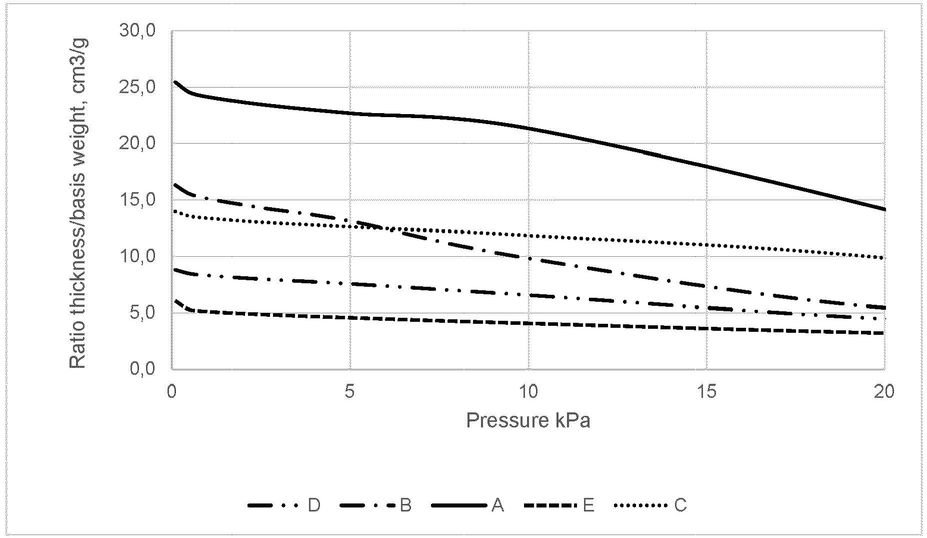

The thickness of the spacer fabric A, B, C, D and E at different applied pressures were as following:

| Spacer fabric | 0.5 kPa | 5.0 kPa | 10 kPa | 20 kPa |
|---|---|---|---|---|
| A | 4.3 | 4.0 | 3.7 | 2.5 |
| B | 2.7 | 2.3 | 1.7 | 1.0 |
| C | 3.0 | 2.8 | 2.7 | 2.2 |
| D | 2.2 | 2.0 | 1.7 | 1.2 |
| E | 3.0 | 2.6 | 2.3 | 1.8 |

The bulk (cm³/g) of the spacer fabric A, B, C, D and E at different applied pressures were as following:

| Spacer fabric | 0.5 kPa | 5.0 kPa | 10 kPa | 20 kPa |
|---|---|---|---|---|
| A | 24.5 | 22.7 | 21.3 | 14.2 |
| B | 15.5 | 13.1 | 9.8 | 5.5 |
| C | 13.6 | 12.6 | 11.8 | 9.8 |
| D | 8.5 | 7.6 | 6.6 | 4.5 |
| E | 5.3 | 4.6 | 4.1 | 3.2 |

The result shows that the spacer fabric named A, B and C have a bulk of 10 g/cm³ or more, at an applied pressure of 0.5 k Pa, and at an applied pressure of 5 kPa, wherein the spacer fabric D and E have a bulk lower than 10 g/cm³ at an applied pressure of 0.5 k Pa and at an applied pressure of 5 kPa. The bulk value is the inversion of the density value, so a spacer fabric with a bulk of 10 cm³/g or more at an applied pressure of 0.5 k Pa and at an applied pressure of 5 kPa, has a density of less than 0.10 g/cm³ at an applied pressure of 0.5 k Pa and at an applied pressure of 5 kPa.

### Example 3 - Liquid retaining capacity measurement

The liquid retaining capacity test measures the amount of liquid held within a test sample after specified times of immersion and vertical drainage. The amount of test liquid that was retained by the test sample was used to calculate and report the liquid retaining capacity in g/cm². The test was performed in a climatized room controlled at 23 C and 50% RH.

The test procedure follows method WSP 010.1.R3 (20) part B (Liquid Absorptive Capacity) with modifications specified as follows. The test liquid is 0.9% saline solution. For the weighing part of the test, no cover glass was used as the test liquid is non-volatile. The sample size of the test sample was 3 cm by 5 cm and the dimension of the wire gauze was 4 cm by 6 cm. The sample was hanged vertically for 60 seconds.

Test samples were prepared as follows. Test samples were removed from a product by using scissors and then a punching tool was used to punch out the sample size of 3 cm by 5 cm.

The immersion and drainage procedure in the method NWSP10.1 was then followed with the modifications previously noted. The dry mass was subtracted from the wet mass and recorded as liquid mass absorbed in grams, then the liquid mass absorbed (g) was divided by the overall area (cm²) of the test sample. A total of three replicates were measured. The arithmetic mean for the liquid retaining capacity in g/cm² was then calculated.

Samples A, D and F were tested. Sample A was a spacer fabric from Muller Textil. Sample D was a reference spacer fabric material from a washable absorbent underwear named Confitex Full Brief Extra, size M. Confitex Full Brief Extra, size M, was a commercially available washable absorbent underwear. Sample F was an absorbent retaining structure of terry material.

The liquid retaining capacity (cm³/g) of the samples A, D and F were as following:

| Sample | Liquid retaining capacity (g/cm²) |
|---|---|
| A | 0.08 |
| D | 0.12 |
| F | 0.24 |

As can be seen from this result, the retaining capacity for sample A, which was a spacer fabric according to the present disclosure was below 0.1 g/cm² and the retaining capacity for sample D, which was a reference spacer fabric, was above 0.1 g/cm². Furthermore, the sample F, which was an absorbent retaining structure of a terry material layer had a retaining capacity above 0.2 g/cm².

To increase the liquid retaining capacity further, the absorbent retaining structure of the present disclosure may comprise more than one liquid retaining layer, such for example two or more layers of the terry material according to sample F, wherein each of the layers have a liquid retaining capacity of 0.24 g/cm². The present disclosure may comprise two or more liquid retaining layers, such for example two or more layers of the terry material according to sample F.

### Example 4 - Acquisition measurement

The acquisition test measures the time required for an absorbent concept to absorb a specified amount of liquid. An acquisition cup was placed on the wearer facing side of the absorbent concept which is the wearer facing side of the liquid permeable layer. A dose of 10 ml is then added with a flow of 20 ml/seconds.

The acquisition time was measured on three absorbent concepts. Each of the absorbent concept 1, 2 and 3, comprising a liquid permeable layer, a liquid acquisition structure and a liquid retaining structure.

The liquid permeable layer in absorbent concept 1, 2, 3 was Sample G. The width of the liquid permeable layer was 6 cm and the length of the liquid permeable layer was 18 cm. The liquid retaining structure in absorbent concept 1, 2, 3 was Sample F. The width of the liquid retaining structure was 6 cm and the length of the liquid retaining structure was 18 cm.

As liquid acquisition structure, the samples A, D and F were tested. The width of liquid acquisition structure was narrower than the liquid permeable layer and the liquid retaining structure. The width of the liquid acquisition structure was 3.5 cm and the length of the liquid acquisition structure was 18 cm.

| Absorbent concept | Liquid acquisition structure | Inlet time (sec) |
|---|---|---|
| 1 | A | 2.0 |
| 2 | D | 3.7 |
| 3 | F | 5.2 |

As can be seen from this result, concept 1 provides a faster acquisition time than concept 2. So, an absorbent concept with a spacer fabric in accordance with the present invention (sample A) provides a faster inlet time compared to an absorbent concept with a reference spacer fabric (sample D).

It can also be seen that absorbent concept 1 and 2 with a spacer fabric provide a faster acquisition time than concept 3 having no spacer fabric. The acquisition structure in absorbent concept 3 was an absorbent terry material, so the same material as the liquid retaining structure.

## Claims

1. Washable absorbent hygienic underwear (1) comprising an outer pant (10) and an absorbent assembly (100), the absorbent assembly (100) and the outer pant (10) having an extension in a longitudinal direction (y), in a transverse direction (x) and in a thickness direction (z) perpendicular to the longitudinal direction (y), and the transverse direction (x), the outer pant (10) comprising a front portion (11), a rear portion (16) and a crotch portion (15), the absorbent assembly (100) being permanently secured in the outer pant (10) and comprising
- a liquid permeable layer (120),
- a liquid impermeable layer (150); and
- an absorbent member (140) located between the liquid permeable layer (120) and the liquid impermeable layer (150), the absorbent member (140) comprising a liquid acquisition structure (141) and a liquid retaining structure (142), the liquid acquisition structure (141) comprising or consisting of a spacer fabric (138) comprising a top web (138a), a bottom web (138b) and an intermediate section (138c) comprising pile filaments extending in the thickness direction (z) and connecting the top web (138a) with the bottom web (138b), the liquid acquisition structure (141) being arranged between the liquid permeable layer (120) and the liquid retaining structure (142); wherein
the spacer fabric (138) completely overlaps with the liquid retaining structure (142) and has an area which is 65% or less of an area of the liquid retaining structure (142), the liquid retaining structure (142) having a density being higher than a density of the spacer fabric (138).

2. Washable absorbent hygienic underwear (1) according to claim 1, wherein the liquid retaining structure (142) has a basis weight which is higher than a basis weight of the spacer fabric (138).

3. Washable absorbent hygienic underwear (1) according to claim 1 or 2, wherein a liquid retaining capacity of the liquid retaining structure (142) is higher than a liquid retaining capacity of the spacer fabric (138).

4. Washable absorbent hygienic underwear (1) according to claim 1, 2 or 3, wherein the absorbent assembly (100) is secured in the outer pant (10) with the liquid acquisition structure (141) being arranged in the crotch portion (15) of the outer pant (10) and/or in the front portion (11) of the outer pant (10).

5. Washable absorbent hygienic underwear (1) according to any one of the preceding claims, wherein, the absorbent assembly (100) and the absorbent member (140) are divided in the longitudinal direction (y) into a front part (111), a rear part (116) and a crotch part (115), the absorbent member (140) having concavely curved side edges (18, 19), the crotch part (115) of the absorbent member (140) being narrower than at least one of the front part (111) and the rear part (116) of the absorbent member (140).

6. Washable absorbent hygienic underwear (1) according to any one of the preceding claims, wherein the crotch part (115) of the absorbent member (140) has a greatest width in the transverse direction (x) which is smaller than a greatest width of the rear part (116) of the absorbent member (140).

7. Washable absorbent hygienic underwear (1) according to any one of the preceding claims, wherein the crotch part (115) of the absorbent member (140) has a greatest width in the transverse direction (x) which is smaller than a greatest width of the front part (111) of the absorbent member (140).

8. Washable absorbent hygienic underwear (1) according to any one of the preceding claims, wherein the front part (111) of the absorbent member (140) has a smaller width in the transverse direction (x) than the rear part (116) of the absorbent member (140), a ratio between the greatest width of the front part (111) of the absorbent member (140) and the greatest width of the rear part (116) of the absorbent member (140) preferably being in the range of from 1:2 to 1:3.

9. Washable absorbent hygienic underwear (1) according to any one of the preceding claims, wherein the spacer fabric (138) in the liquid acquisition structure (141) has a length along a longitudinal centerline (Lc) through the absorbent hygienic underwear (1) which is smaller than a length of the absorbent member (140) along the longitudinal centerline (Lc), a ratio between the length of the spacer fabric (138) in the liquid acquisition structure (141) and the length of the absorbent member (140) as measured along the longitudinal centerline (138) being in the range of from 2.5:4 to 3:4.

10. Washable absorbent hygienic underwear (1) according to any one of the preceding claims, wherein the spacer fabric (138) in the liquid acquisition structure (141) preferably has a front end edge (14') positioned at a front end edge (14) of the absorbent member (140) and a rear end edge (17') positioned at a distance (d) from the rear end edge (17) of the absorbent member (140), the distance (d) preferably being at least 15% of a length (lₐₘ) of the absorbent member (140) as measured along the longitudinal centerline (L_{C}), more preferably in the range of from 15% to 40%, or 20% to 30%.

11. Washable absorbent hygienic underwear (1) according to any one of the preceding claims, wherein an area of the spacer fabric (138) is 60% or less of an area of the absorbent assembly (100), such as 50% or less of the area of the absorbent assembly (100), such as 40% or less of the area of the absorbent assembly (100).

12. Washable absorbent hygienic underwear (1) according to any one of the preceding claims, wherein the spacer fabric (138) is secured to at least one other component in the absorbent assembly (100) by means of a flat seam, such as a flatlock stitch.

13. Washable absorbent hygienic underwear (1) according to claim 12, wherein the spacer fabric (138) is secured to at least one other component in the absorbent assembly (100) by a flatlock stitch in class 600 according to the international standard ISO 4915, preferably by a flatlock stitch type 607 according to the international standard ISO 4915.

14. Washable absorbent hygienic underwear (1) according to claim 12 or 13, wherein the spacer fabric (138) is secured to the at least one other component in the absorbent assembly (100) along a front-end edge (14') and a rear-end edge (17') of the spacer fabric (138) and/or along a first side edge (18') and a second side edge (19') of the spacer fabric (138).

15. Washable absorbent hygienic underwear (1) according to claim 12, 13 or 14, wherein the spacer fabric (138) is secured to the liquid retaining structure (142).

16. Washable absorbent hygienic underwear (1) according to any one of the preceding claims, wherein the liquid acquisition structure (141) consists of a single layer of spacer fabric (138) or of a layer of spacer fabric (138) and one or more additional material layers.

17. Washable absorbent hygienic underwear (1) according to any one of the preceding claims, wherein the spacer fabric (138) in the liquid acquisition structure (141) has a bulk of more than 10 cm³/g at an applied pressure of 5 kPa.

18. Washable absorbent hygienic underwear (1) according to any one of the preceding claims, wherein the layer of spacer fabric (138) is in direct contact with the liquid retaining structure (142).

19. Washable absorbent hygienic underwear (1) according to any one of the preceding claims, wherein the spacer fabric (138) has a density of less than 0.1 g/cm³, optionally between 0.05 and 0.09 g/cm³.

20. Washable absorbent hygienic underwear (1) according to any one of the preceding claims, wherein the density of the liquid retaining structure (142) is 20% higher than the density of the spacer fabric (138), or more than 50% higher than the density of the spacer fabric (138).

21. Washable absorbent hygienic underwear (1) according to any one of the preceding claims, wherein the liquid retaining structure (142) consists of a single material layer or of 2 or more individual material layers.

22. Washable absorbent hygienic underwear (1) according to any one of the preceding claims, wherein the washable absorbent hygienic underwear (1) is in the form of a female incontinence pant.
